# EUROPEAN PATENT SPECIFICATION

(11) **EP 1 299 114 B1**
(45) Date of publication and mention of the grant of the patent: **10.08.2005**
(21) Application number: 01913407.1
(22) Date of filing: 20.02.2001
(51) Int. Cl.: A61K 38/51, A61K 38/48, A61K 31/196, A61K 31/355, A61K 9/00, A61P 29/00

(54) **DICLOFENAC PHARMACEUTICAL COMPOSITION BASED ON VITAMIN-E, PAPAIN AND HYALURONIDASE**
PHARMAZEUTISCHE DICLOFENAC-VERBINDUNG BASIEREND AUF VITAMIN E, PAPAIN UND HYALURONIDASE
COMPOSITION PHARMACEUTIQUE DE DICLOFENAC A BASE DE VITAMINE E, PAPAINE ET HYALURONIDASE

(30) Priority: 21.02.2000 BR 0001144
(43) Date of publication of application: 09.04.2003
(73) Proprietor: Topic Empreendimentos e Participaçoes S/C Ltda., CEP-06453-000 Barueri, SP (BR)
(72) Inventor: RIBEIRO SANTANA, Cristiano, Alberto, CEP-01250-000 Sao Paulo, SP (BR); DE NUCCI, Gilberto, CEP-13092-320 Campinas, SP (BR); FALCI, Marcio, CEP-05641-900 Sao Paulo, SP (BR)
(74) Representative: Prato, Roberto
(86) International application number: PCT/BR2001/000019
(87) International publication number: WO 2001/060399

(56) References cited:
- EP-A- 0 788 794
- EP-A1- 0 475 160
- EP-A2- 0 283 434
- WO-A-94/22423
- BR-A- 9 801 985
- FR-A- 2 448 903

## Description

The present invention relates to a new DICLOFENAC PHARMACEUTICAL COMPOSITION BASED ON VITAMIN-E, PAPAIN AND HYALURONIDASE. A Said composition is preferably of topical application, non-toxic and features a high rate of penetration through the skin.

### DESCRIPTION OF THE INVENTION

The skin permeability varies according to the region of the body, being the skin folds and the face those that present the highest absorption rate. A product applied over the skin will present a longer period of contact and percutanial absorption.

According to the classic book "Histologia dos epitélios", by Walter A. Hadler and Sineli R. Silveira, Editora Campus, Campinas, 1993, it is considered that: "bearing in mind the general morphological characteristics and the speciolized functions that they perform, the epitelium cells are predominantly classified into two categories, which correspond to two epitelium classes: coating epitelium cells and secreting epitelium cells. The cells of these two classes mix with each other to constitute, respectively, the coating epiteliums and the secreting epiteliums, each one of them performing specific functions that are inherent to them. Such division is also fundamented in the distribution of these two classes of epitelium in the organism, which altough wide is is distinctive for both. With the purpose of forming the coating epiteliums the epitelium cells associate side-by-side, so as to originate "membranes" or layers superimposed over the base membrane, which function is to coat surfaces. On the contrary, the secreting cells unite to form organized functional units, better suited for performing their specialized function, related to the secretion products synthesys; thus are constituted the secreting units. The coating epiteliums are defined as living membranes, usually featuring a discontinuity, that isolate the organism from the environment, separating the internal media from the external one. Furthermore, these epiteliums isolate from each other the various internal media compartments, among which are the intravascular compartment, the serum compartment and several others. Among the various functions performed by the coating epiteliums some are performed by specialized variants that are specifically adapted to perform one or more functions. Others are incorporated as general functions presented without distinction by every coating epitelium cell. The coating epitelium cell, in the same way as most of the living cells, passively absorbs water and electrolytes and eliminates them actively; this function is well developed in the epitelium cells. On that account it is very important to observe that generally it is understood as absorption the penetration of solutions through the cells plasmatic membrane. However two different specific forms of absorption must be distinguished from one another: the passive absorption, that occurs according to the osmotic laws, and the active absorption, that entails the effective participation of the epitelium cell and that does not follow such physic laws. On the other hand it must be considered that every single substance that penetrates the interior of a multi-cellular organism, or else is excreted or elliminated, must cross at least one coating epitelium, because every superior organism is penetrated internally and externally by epiteliums. It must also be observed that the coating epiteliums, altough continuously covering and protecting those surfaces it coats, are not impervious at all; that is why they do not behave as inert "membranes". On the contrary, they allow for the exchange of gases, water, several kinds of electrolytes and certain other solutes between the internal and the external media, or between the various internal compartments, which characterizes its permeability. The coating epitelium cells limit in a controlled and selective way the permeability of the respective epiteliums, with the purpose of protecting the organism and still participate of the control of its homeostasis. In order to perform such function the epiteliums are organized and arrange their cells in a special form, in order to build up coatings which cells abbut the base membrane and are united with each other by means of intracellular junctions; in turn the cells are coated by the plasmatic membrane, which features special characteristics, and by the glicochalice, both able to express well defined functional properties. The functional characteristics expressed by the plasmatic membrane portion that coats the cells apical surface are different from those expressed by the portion situated in its basal or basolateral face; such differences, which occur mainly on the funsctional aspect, contribute for the remarkable degree of polarization expressed by the coating epitelium cells. The prime function performed by the coating epiteliums correspond essentialy to the protection rendered to the surface that they coat, characterizing their protective coating function. Such function features a special characteristic, being a coating that, besides offering mechanical, physical and chemical protection to the coated surface, is not inert. The coating epiteliums are pervious, which allows for the controlled and selective passage of several products through its wall.

It is fairly well demonstrated that the permeability degree influences strongly the function performed by the coating epiteliums:
1) wide permeability;
2) reduced permeability and
3) absence of permeability.

The purpose is to prove through the formulation that there is an intense metabolic exchange demonstrating that the epithelium actuates on the transfer of metabolytes. This penetration of substances is complete and gradual and trespasses these epithelium layers until it penetrates the small blood vessels, reaching the circulatory current.

There is a description of the molecules to estimate the coating epitheliums permeability. Ex.: Hemoglobin, Ferritin, Lipoproteins and enzymes.

Is is also known the transcitose on the transposition of the epiteliums by the macro and micro molecules until the vascular eye depending of their association.

The object of the present invention is a DICLOFENAC PHARMACEUTICAL COMPOSITION BASED ON VITAMIN-E, PAPAIN AND HYALURONIDASE.

Advantageously, the present invention commends the diclofenac pharmaceutical composition based on vitamin E, papain and hyaluronidase, notably in topical applications, with a high rate of penetration through the skin.

More advantageously the pharmaceutical composition according to the present invention, comprises a gel, cream or gel cream form of diclofenac.

Nevertheless, more advantageously, the diclofenac could be used, notably of sodium or potassium. The diclofenac is an anti-inflammatory drug, non steroidal, with analgesic and anti-pyretic properties. Its chemical formulation is C₁₄H₁₀NO₂CL₂X (being X a radical, in this case ethyl ammonia) its structural elements include an phenylacceptic acidic group, a secondary amine and a phenyl ring with atoms of chlorine in the ortho-position. The chlorine atoms cause maximum torsion of the phenyl ring. Diclofenac is a weak acid (pka = 4,0) featuring a partition coefficient of 13.0 (octanol/tampoom phosphate.ph=7,4). Diclofenac's solubility under physiologic conditions varies from 17,8 mg/l (neutral ph) to less than 1 mg/l (acidic ph) (KUROWSKI ET/AL, 1994).

Diclofenac inhibits the activity of cycloxygenase, with reduction of the production of tissue prostaglandines such as PGF2 and PGE2. its anti-inflammatory effect, ascertained in the model of arthritis induced by adjuvant, is higher than that of aspirin.

The use of hyaluronidase as a diffusion factor is already well established, and is commercially available in the injectable form. The enzyme is extracted from bovine testicles arid commercialized in the lyophilized form by APSEN DO BRASIL LTDA laboratories (hyalozime 2.000 UTR and 20.000 UTR). Similarly the use of Vitamin E (alpha tocopherol) as an anti-oxidizer is already common in several vitamin complexes. Papain, provided by the fruit of *Carica papaya,* has been used in several areas of medical science, as an anti-aggregator for platelets (METZIG et al, 1999), in the catalysis for the synthesis of peptides (STEHLE et al, 1990) and in the treatment of abscesses. (UDOD et al., 1989).

The present technique is required as a composition of DICLOFENAC aggregated comprising the following formulation:

| | |
|---|---|
| PAPAIN | 0.1 to 15% |
| HYALURONIDASE | 50 to 900 utr/mg |
| VITAMIN E | 10 to 200mg |

This technique was proofed through studies performed with 08 outpatients, in 02 distinct sessions of double blind analysis. The delimited area measured 15x10 cm with the application of gel, after 15 minutes the measurements were started through liquid chromatography coupled to mass spectometry.

The comparison of a confirm cream with the same active substance, for the purpose of calibration, yielded the following results regarding the cream according to the present invention, with an analytical type of equal absorption area:

### Example 1 (1% Diclofenac)

| | |
|---|---|
| PAPAIN | 0.002g |
| HYALURONIDASE | 220 utr/mg |
| VITAMIN-E | 0.020g |

CONFIRM CREAM = Area 131 vol. 0.54 = 8 hours
TESTED CREAM (INVENTION) = Area 131 vol. 0.54 = 2 hours

### Example 2 (1% Diclofenac)

| | |
|---|---|
| PAPAIN | 0,001g |
| HYALURONIDASE | 75 utr/mg |
| VITAMIN-E | 0,010g |

CONFIRM CREAM = Area 131 vol. 0.54 = 8 hours
TESTED CREAM (INVENTION) = Area 131 vol. 0.54 = 3.20 hours

### Example 3 (1% Diclofenac)

| | |
|---|---|
| PAPAIN | 0,003g |
| HYALURONIDASE | 94 utr/mg |
| VITAMIN-E | 0,014g |

CONFIRM CREAM = Area 131 vol. 0.54 = 8 hours
TESTED CREAM (INVENTION) = Area 131 vol. 0.54 = 2,50 hours

### Example 4 (1% Diclofenac)

| | |
|---|---|
| PAPAIN | 0,004g |
| HYALURONIDASE | 90 utr/mg |
| VITAMIN-E | 0,016g |

CONFIRM CREAM = Area 131 vol. 0.54 = 8 hours
TESTED CREAM (INVENTION) = Area 131 vol. 0.54 = 1,10 hours

### Example 5 (1% Diclofenac)

| | |
|---|---|
| PAPAIN | 0,005g |
| HYALURONIDASE | 100 utr/mg |
| VITAMIN-E | 0,017g |

CONFIRM CREAM = Area 131 vol 0.54 = 8 hours
TESTED CREAM (INVENTION) = Area 131 vol. 0.54 = 1,00 hours

### Example 6 (1% Diclofenac)

| | |
|---|---|
| PAPAIN | 0,008g |
| HYALURONIDASE | 99 utr/mg |
| VITAMIN-E | 0,020g |

CONFIRM CREAM = Area 131 vol. 0.54 = 8 hours
TESTED CREAM (INVENTION) = Area 131 vol. 0.54 = 50 minutes

## Claims

1. **"DICLOFENAC PHARMACEUTICAL COMPOSITION BASED ON VITAMIN-E, PAPAIN AND HYALURONIDASE" characterized by** the fact that it comprises the following formulation:
| | |
|---|---|
| PAPAIN | 0.1 to 15% |
| HYALURONIDASE | 0,2085 to 3,75 mg/g (50 to 900 TRU/mg) |
| VITAMIN E ..... | 10 to 2000 mg |

2. **"DICLOFENAC PHARMACEUTICAL COMPOSITION BASED ON VITAMIN-E, PAPAIN AND HYALURONIDASE",** according to claim 1, **characterized by** the fact that said composition comprises diclofenac of sodium and/or potassium.

3. **"DICLOFENAC PHARMACEUTICAL COMPOSITION BASED ON VITAMIN-E, PAPAIN AND HYALURONIDASE",** according to claim 1 or 2, **characterized by** the fact that said composition comprises the form of gel, cream or cream gel.

4. **"DICLOFENAC PHARMACEUTICAL COMPOSITION BASED ON VITAMIN-E, PAPAIN AND HYALURONIDASE",** according to claim 3, **characterized by** the fact that said composition is applied topically.

5. **"DICLOFENAC PHARMACEUTICAL COMPOSITION BASED ON VITAMIN-E, PAPAIN AND HYALURONIDASE",** according to claim 2, **characterized by** the fact that diclofenac of sodium and/or potassium is present in said composition in a quantity greater than 0.03%.

## Patentansprüche

1. "Pharmazeutische Diclofenaczusammensetzung, basierend auf Vitamin E, Papain und Hyaluronidase", **gekennzeichnet durch** die Tatsache, dass diese die folgende Formulierung aufweist:
| | |
|---|---|
| Papain | 0,1 bis 15 % |
| Hyaluronidase | 0,2085 bis 3,75 mg/g (50 bis 900 TRU/mg) |
| Vitamin E | 10 bis 2000 mg |

2. "Pharmazeutische Diclofenaczusammensetzung, basierend auf Vitamin E, Papain und Hyaluronidase" nach Anspruch 1, **gekennzeichnet durch** die Tatsache, dass die Zusammensetzung Diclofenac-Natrium- und / oder Diclofenac-Kalium aufweist.

3. "Pharmazeutische Diclofenaczusammensetzung, basierend auf Vitamin E, Papain und Hyaluronidase" nach Anspruch 1 oder 2, **gekennzeichnet durch** die Tatsache, dass die Zusammensetzung die Form eines Gels, einer Creme oder eines Cremegels aufweist.

4. "Pharmazeutische Diclofenaczusammensetzung, basierend auf Vitamin E, Papain und Hyaluronidase" nach Anspruch 3, **gekennzeichnet durch** die Tatsache, dass die Zusammensetzung topisch angewendet wird.

5. "Pharmazeutische Diclofenaczusammensetzung, basierend auf Vitamin E, Papain und Hyaluronidase" nach Anspruch 2, **gekennzeichnet durch** die Tatsache, dass Diclofenac-Natrium- und / oder Diclofenac-Kalium der Zusammensetzung in einer Menge größer als 0,03 % vorhanden ist.

## Revendications

1. Composition pharmaceutique de diclofénac à base de vitamine E, de papaïne et de hyaluronidase, **caractérisée en ce qu'**elle comprend la formulation suivante :
| | |
|---|---|
| Papaïne | 0,1 à 15 % |
| hyaluronidase | 0,2085 à 3,75 mg/g (50 à 900 TRU/mg) |
| vitamine E | 10 à 2000 mg |

2. Composition pharmaceutique de diclofénac à base de vitamine E, de papaïne et de hyaluronidase, selon la revendication 1, **caractérisée en ce que** ladite composition comprend du diclofénac sodique et/ou potassique.

3. Composition pharmaceutique de diclofénac à base de vitamine E, de papaïne et de hyaluronidase, selon la revendication 1 ou 2, **caractérisée en ce que** ladite composition a la forme d'un gel, d'une crème ou d'un gel-crème.

4. Composition pharmaceutique de diclofénac à base de vitamine E, de papaïne et de hyaluronidase, selon la revendication 3, **caractérisée en ce que** ladite composition est appliquée par voie topique.

5. Composition pharmaceutique de diclofénac à base de vitamine E, de papaïne et de hyaluronidase, selon la revendication 2, **caractérisée en ce que** le diclofénac sodique et/ou potassique est présent dans ladite composition en une quantité supérieure à 0,03 %.
